**Europäisches Patentamt**

**European Patent Office**

**Office européen des brevets**

(19)

(11) Veröffentlichungsnummer: **0 008 445**

**B1**

(12) **EUROPÄISCHE PATENTSCHRIFT**

(21) Anmeldenummer: **79103006.7**

(22) Anmeldetag: **17.08.79**

(51) Int. Cl.³: **C 07 C 63/06, C 07 C 51/56**

(54) Verfahren zur Herstellung von Benzoesäureanhydrid

(30) Priorität: **28.08.78 DE 2837457**

(43) Veröffentlichungstag der Anmeldung:
**05.03.80 Patentblatt 80/05**

(45) Bekanntmachung des Hinweises auf die Patenterteilung:
**10.12.80 Patentblatt 80/25**

(84) Benannte Vertragsstaaten:
**BE CH DE FR GB IT NL**

(56) Entgegenhaltungen:
**DE - C - 368 340**
**US - A - 3 438 882**

(73) Patentinhaber: **BAYER AG**
**Zentralbereich Patente, Marken und Lizenzen**
**D - 5090 Leverkusen 1, Bayerwerk (DE)**

(72) Erfinder: **Goliasch, Karl, Dr.**
**Drosselweg 37**
**D - 5060 Bergisch-Gladbach 2 (DE)**
**Müller, Herbert, Dr.**
**Weismantelweg 11**
**D - 5000 Köln 91 (DE)**
**Pelster, Heinrich, Dr.**
**Auf dem Heidgen 23**
**D - 5068 Odenthal (DE)**

Courier Press, Leamington Spa, England.

Verfahren zur Herstellung von Benzoesäureanhydrid

Die Erfindung betrifft ein Verfahren zur Herstellung von Benzoesäureanhydrid aus Benzotrichlorid und Benzoesäure.

Es ist bekannt, Benzoesäureanhydrid durch Umsetzung von Benzotrichlorid mit Natriumbenzoat bei Temperaturen von 160 bis 170°C herzustellen (DE-PS 368 340, Beispiel 4).

Diese Verfahrensweise hat den Nachteil, daß im Anschluß an die Reaktion Benzoesäureanhydrid von den entstandenen Salzen abgetrennt werden muß. Die Abtrennung des Benzoesäureanhydrids von den Salzen erfordert einen erheblichen technischen Aufwand, da die Salze in feinverteilter, schwer filtrierbarer Form anfallen.

Auch die Abtrennung des Benzoesäureanhydrids durch Destillation ist wenig wirtschaftlich, da die Destillation im Vakuum bei Temperaturen von ca. 200°C erfolgt und wegen der im Destillationssumpf enthaltenen, Korrosion hervorrufenden Salze in Spezialapparaturen durchgeführt werden muß.

Ebensowenig wirtschaftlich ist die Extraktion des Reaktionsgemisches zur Gewinnung von Benzoesäureanhydrid, da dafür zusätzliche Apparaturen und größere Mengen Lösungsmittel erforderlich sind. Zusätzlich muß der Extrakt von den Salzen abgetrennt werden, wodurch sich die oben geschilderten Probleme ergeben.

Es wurde nun ein Verfahren zur Herstellung von Benzoesäureanhydrid gefunden, das dadurch gekennzeichnet ist, daß man Benzotrichlorid mit Benzoesäure im Molverhältnis von etwa 1:2 bis etwa 1:3 bei Temperaturen im Bereich von etwa 100 bis etwa 200°C umsetzt.

Bei einer bevorzugten Ausführungsform des erfindungsgemäßen Verfahrens beträgt das Molverhältnis von Benzotrichlorid zu Benzoesäure etwa 1:2,5. Die Umsetzung findet bei Reaktionstemperaturen im Bereich von etwa 140 bis etwa 180°C statt.

Nach Beendigung der Reaktion wird das Benzoesäureanhydrid von noch nicht restlos umgesetzter Benzoesäure und noch nicht vollständig umgesetztem Benzotrichlorid sowie von in geringen Mengen als Nebenprodukt gebildetem Benzoylchlorid durch Andestillieren bei Temperaturen im Bereich von ca. 120 bis 200°C, bevorzugt 140 bis 180°C, und Drucken im Bereich von ca. 10 bis 200 mbar, bevorzugt 20 bis 100 mbar, abgetrennt.

Dabei verbleibt Benzoesäureanhydrid als hochsiedender Destillationsrückstand in reiner Form ($\geq$ 98% Reinheit) im Destillationsgefäß. Das gesamte abgetrennte Destillatgemisch, bestehend aus Benzoesäure, Benzotrichlorid und Benzoylchlorid, wird zurückgeführt und dem Ausgangsreaktionsgemisch, d.h. einem neuen Ansatz, wieder zugegeben. Auf diese Weise werden die Einsatzkomponenten vollständig umgesetzt und eine nahezu quantitative Ausbeute an Benzoesäureanhydrid erzielt.

Zur Beschleunigung der Reaktion und zur Vervollständigung des Umsatzes kann in das Reaktionsgemisch Inertgas eingeleitet werden. Dabei geht man im allgemeinen so vor, daß die Inertgasmenge währen der Einleitungsdauer allmählich gesteigert wird. Die Endmenge an Inertgas kann dabei das etwa 2-fache bis etwa 10-fache, bevorzugt 4-fache bis 8-fache, der Ausgangsmenge an Inertgas betragen. Gleichzeitig mit dem Einleiten von Inertgas wird die Reaktionstemperatur allmählich von etwa 120 bis auf etwa 180°C gesteigert.

Als Inertgas kommt bevorzugt Stickstoff in Frage.

Dei Menge Inertgas, die in das Reaktionsgemisch eingeleitet wird, richtet sich nach der Menge der Einsatzprodukte und der Reaktordimension und beträgt im allgemeinan ca. 1 bis 10 m$^3$, bevorzugt 3 bis 8 m$^3$, pro Stunde und pro m$^3$ Reaktionsvolumen.

Den gleichen Effekt, d.h. Beschleunigung der Reaktion und Vervollständigung des Umsatzes, kann man erreichen, wenn man anstatt Einleiten von Inertgas dem Reaktionsgemisch inerte organische Lösungsmittel zugibt. Dabei werden vorteilhafterweise die inerten organsichen Lösungsmittel nicht zu Beginn der Reaktion, sondern erst nach Erreichen von etwa 60% Umsatz und Abklingen der ersten kräftigen Chlorwasserstoffentwicklung, zugesetzt. Es ist zweckmäßig, das inerte organische Lösungsmittel in mehreren kleinen Anteilen oder kontinuierlich dem Reaktionsgemisch zuzugeben, derart, daß das Reaktionsgemisch unter Einhaltung von etwa 150 bis etwa 180°C Sumpftemperatur ständig am Rückfluß gehalten wird.

Als inerte, organische Lösungsmittel kommen solche in Frage, die unter Reaktionsbedingungen verdampfbar sind.

Zum Beispiel können gegebenenfalls durch Halogen und/oder durch Alkylreste mit 1 bis 10, bevorzugt 1 bis 8, Kohlenstoffatomen ein- oder mehrfach substituierte aliphatische, cycloaliphatische, araliphatische oder aromatische Kohlenwasserstoffe mit bis zu 12 Kohlenstoffatomen, bevorzugt bis zu 8 Kohlenstoffatomen, eingesetzt werden.

Als Halogene seien genannt: Fluor, Chlor, Brom bevorzugt Fluor, Chlor.

Als gegebenenfalls substituierte aliphatische Kohlenwasserstoffe seien z.B. genannt:

Pentan, Hexan, Heptan, Dichlormethan, Trichlormethan, Tetrachlormethan, 1,2-Dichloräthan, 1,1,1-Trichloräthan, 1,1,2-Trichloräthylen, 1,2-Dichlorbutan, bevorzugt: Hexan;

als gegebenenfalls substituierte cycloaliphatische Kohlenwasserstoffe:

Cyclohexan, Methylcyclohexan, bevorzugt: Cyclohexan;

als gegebenenfalls substituierte araliphatische Kohlenwasserstoffe:

Toluol, Xylol, Trimethylbenzol, Äthylbenzol, bevorzugt: Toluol;

als gegebenenfalls substituierte aromatische Kohlenwasserstoffe:

Benzol, Fluorbenzol, Chlorbenzol, 1,2-Dichlorbenzol, 1-Chlortoluol, bevorzugt: Chlorbenzol.

In das erfindungsgemäße Verfahren werden bevorzugt chlorierte aliphatische oder aromatische Kohlenwasserstoffe wie Dichlormethan, Chlorbenzol, Dichlorbenzol als inerte organische Lösungsmittel eingesetzt.

Die inerten organischen Lösungsmittel können einzeln oder im Gemisch untereinander dem Reaktionsgemisch zugegeben werden. Es ist auch möglich, daß man in das mit inerten organischen Lösungsmitteln versetzte Reaktionsgemisch zusätzlich Inertgas einleitet.

Die Menge der zugesetzten inerten organischen Lösungsmittel richtet sich nach der Art der Lösungsmittel und kann leicht durch Vorversuche ermittelt werden.

Eine Beschleunigung der Reaktion kann auch durch Anlegen eines leichten Vakuums erzielt werden. Dabei wird bei etwa 945 bis 500 mbar (700 bis 380 Torr), bevorzugt 675 bis 530 mbar (500 bis 400 Torr) gearbeitet.

Das erfindungsgemäße Verfahren kann sowohl diskontinuierlich als auch kontinuierlich durchgeführt werden.

In einer bevorzugten Ausführungsform des erfindungsgemäßen Verfahrens wird in einem Rührwerkskessel Benzotrichlorid mit Benzoesäure im Molverhältnis 1:2,5 unter Rühren und Erhitzen auf etwa 140 bis 150°C zur Reaktion gebracht. Die Aufheizrate wird so gewählt, daß eine weitgehend gleichmäßige, gegen Reaktionsende schwächer werdende Chlorwasserstoffentwicklung stattfindet. Nach Abklingen der ersten Chlorwasserstoffentwicklung wird Stickstoff in das Reaktionsgemisch eingeleitet. Dabei geht man im allgemeinen so vor, daß die Stickstoffmenge wären der Einleitungsdauer allmählich gesteigert wird. Die Endmenge an Stickstoff kann das 2- bis 10-fache der Ausgangsmenge an Stickstoff betragen.

Nachdem 2,5 Mole Chlorwasserstoff, bezogen auf eingesetztes Benzotrichlorid, freigesetzt worden sind, wird die Reaktion abgebrochen. Anschließend wird das Reaktionsgemisch durch Andestillieren im Vakuum bei etwa 20 bis 100 mbar und etwa 140 bis 180°C aufgearbeitet. Dabei werden die nicht umgesetzte Benzoesäure zusammen mit restlichem Benzotrichlorid und Benzoylchlorid von Benzoesäureanhydrid abgetrennt, das in reiner Form als Rückstand verbleibt.

Das nach dem erfindungsgemäßen Verfahren aus Benzotrichlorid und Benzoesäure hergestellte Benzoesäureanhydrid fällt als undestillierte Rohware in hoher Reinheit ($\geq$ 98% ig) und nahezu farblos an. Die Ausbeute beträgt etwa 95% der Theorie (bezogen auf Benzotrichlorid). Das Benzoesäureanhydrid enthält weniger als 1% Benzoesäure und weniger als 0,2% Benzoylchlorid (berechnet aus einem verseifbaren Cl-Gehalt von weniger als 0,05%).

Die Vorteile des erfindungsgemäßen Verfahrens sind vor allem in dem nahezu quantitativen Umsatz des Benzotrichlorids zu Benzoesäureanhydrid sowie in der hohen Reinheit, mit der Benzoesäureanhydrid ohne vorherige Destillation erhalten wird, zu sehen.

Des weiteren kann bei der Durchführung des erfindungsgemäßen Verfahrens auf die Verwendung von teuren Spezialapparaturen und auf die Durchführung aufwendiger Trennoperationen verzichtet werden, da beim erfindungsgemäßen Verfahren die nicht umgesetzten Ausgangsstoffe von Benzoesäureanhydrid abgetrennt werden.

Benzoesäureanhydrid ist beispielsweise ein wertvolles Zwischenprodukt für die Herstellung von Benzoylcyanid, das z.B. durch Umsetzung mit Blausäure entsteht und das zur Herstellung von Pflanzenschutzmitteln Verwendung findet (vgl. DE-OS 2 224 161).

Das erfindungsgemäße Verfahren wird durch die folgenden Beispiele beschrieben, ohne es jedoch auf diese Beispiele einzuschränken.

### Beispiel 1

In einem emaillierten heiz- und kühlbaren Rührwerkskessel mit 1 m³ Rauminhalt mit geeigneten Destillationseinrichtungen aus Glas werden ca. 500 kg Benzoesäure (4,10 Mol) und ca. 320 kg Benzotrichlorid (1,64 Mol) durch Erhitzen auf 140 bis 150°C zur Reaktion gebracht. Die Aufheizrate pro Zeiteinheit wird nach der Chlorwasserstoff-Entwicklung gesteuert. Sie dauert etwa 5 Stunden. Der entweichende Chlorwasserstoff wird einer geeigneten Absorptionsanlage zur Herstellung von Salzsäure zugeführt.

Dem Folgeansatz wird außerdem das Destillat des Voransatzes bestehend aus ca. 200 bis 300 kg einer Mischung aus ca. 10 bis 30% Benzotrichlorid, ca. 50 bis 80% Benzoylchlorid und ca. 5 bis 10% Benzoesäure hinzugefügt. Dem neuen Ansatz wird soviel Benzoesäure und Benzotrichlorid zugefügt, daß das Molverhältnis Benzoesäure zu Benzotrichlorid etwa 1:2,5 beträgt. Hierbei wird angenommen, daß der Cl-Gehalt des Destillates des Voransatzes ausschließlich als Benzotrichlorid vorliegt.

Nach 3 bis 4 Stunden Reaktionsdauer bei ca. 140°C läßt die Chlorwasserstoffentwicklung nach. Über ein Tauchrohr wird nun Stickstoff eingeblasen. Die Stickstoffmenge wird von anfangs ca. 1 m³ pro Stunde innerhalb von 5 Stunden auf 5 m³ pro Stunde gesteigert.

Innerhalb von ca. 10 Stunden wird die Hauptmenge an Chlorwasserstoff freigesetzt. Der Reaktionsverlauf wird analytisch verfolgt. Die Reaktion wird dann abgebrochen, nachdem mindestens 2,5 Mole HCl, bezogen auf eingesetztes Benzotrichlorid, freigesetzt worden sind.

Nachdem die Reaktion abgebrochen worden ist, wird das Benzoesäureanhydrid von noch nicht restlos umgesetzter Benzoesäure und noch nicht vollständig umgesetztem Benzotrichlorid sowie von als Nebenprodukt gebildetem Benzoylchlorid durch Destillation bei Temperaturen im Bereich von 140 bis 180°C und Drucken im Bereich von 20 bis 100 mbar abgetrennt. Das Benzoesäureanhydrid verbleibt dabei als hochsiedender Destillationsrückstand in reiner Form (≧ 98 %ig) im Destillationsgefäß. Das gesamte abgetrennte Destillatgemisch bestehend aus Benzoesäure, Benzotrichlorid und Benzoylchlorid wird, wie zuvor beschrieben, zurückgeführt und einem neuen Reaktionsansatz wieder zugegeben.

Nach Ausführung von 16 Ansätzen in der vorbeschriebenen Weise sind insgesamt 5198 kg Benzotrichlorid und 8806 kg Benzoesäure verbraucht worden und 10 376 kg Benzoesäureanhydrid mit ≧ 98 %iger Reinheit als Destillationsrückstand erhalten worden.

Als Nebenprodukt wurden dabei 844 kg Destillat (Vorläufe) ausgewogen, die bei einem Chloridgehalt (bezogen auf reines Benzotrichlorid) von ca. 30% 465 kg Benzotrichlorid und 379 kg Benzoesäure entsprechen. Unter entsprechender Einsatzkorrektur errechnet sich hieraus eine Ausbeute von 95 % der Theorie, bezogen auf Benzotrichlorid und 100 % der Theorie, bezogen auf Benzoesäure.

### Beispiel 2

In einem 1 l Dreihalskolben mit Rührer, Dosiertrichter und Rückflußkondensator (aufgebaut auf einer geeigneten Laborwaage) wurden 391 g Benzotrichlorid (2 Mol) und 610 g Benzoesäure (5 Mol) vorgelegt. Bei diesem Versuch wurde der Umsatzverlauf aufgrund des durch die Chlorwasserstoffentwicklung bedingten Gewichtsverlustes verfolgt. Als 100 %iger Umsatz wurde ein Gewichtsverlust von 182,5 g gewertet.

Folgende Vergleichsversuche wurden durchgeführt:
1) Reaktion ohne Stickstoffeinleitung;
2) Reaktion mit Stickstoffeinleitung;
3) Reaktion unter Zusatz von Chlorbenzol (nach 3 Stunden Reaktionsdauer allmähliche portionsweise Zugabe von insgesamt 193 g Chlorbenzol).

Die aus den Gewichtsverlusten ermittelten Zeitumsatzkurven sind in der Abbildung dargestellt.

Die Versuche zeigen, da durch Chlorbenzolzugabe gegenüber der Stickstoffeinleitung die Reaktionszeit nochmals verkürzt und die Umsätze erhöht werden können. Hierbei wird das Chlorbenzol vorteilhafterweise nicht bereits vor dem Start der Reaktion, sondern erst nach 2/3 des Umsatzes in mehreren Anteilen nach und nach zugesezt, so daß der Ansatz bei ca. 180°C Sumpftemperatur stetig am Rückfluß siedet.

## Zeit / Umsatz Kurve

### Beispiel 3

In einem 2 l Dreihalskolben mit Rührer und Rückflußkühler wurden 586,5 g Benzotrichlorid (3 Mol) und 722 g Benzoesäure (6 Mol) eingewogen und auf 150°C geheizt, wobei ab ca. 110°C Chlorwasserstoff-Abspaltung beginnt. Nach 5 Stunden wird auf 180°C aufgeheizt und diese Temperatur 3 Stunden lang gehalten. Danach wird durch Andestillation in Vakuum (140 - 180°C, 200 - 20 mbar) von nicht umgesetztem Benzotrichlorid und Benzoesäure sowie vom Nebenprodukt Benzoylchlorid abgetrennt (960 g Destillat). Als Destillationsrückstand verbleiben 703,9 g Benzoesäureanhydrid (98,2 %ig, Fp. 39 - 40°C), d.s. 77,8 % Umsatz.

Bei wiederholter Versuchsdurchführung unter Hinzufügen des Destillats aus den Vorversuchen zum jeweils nächsten Einsatz errechnet sich eine praktisch quantitative Ausbeute, bezogen auf Benzoesäure.

### Patentansprüche

1. Verfahren zur Herstellung von Benzoesäureanhydrid, dadurch gekennzeichnet, daß man Benzotrichlorid mit Benzoesäure im Molverhältnis von 1:2 bis 1:3 bei Temperaturen im Bereich von 100 bis 200°C umsetzt.

2. Verfahren nach Anspruch 1, dadurch gekennzeichnet, daß das Molverhältnis von Benzotrichlorid zu Benzoesäure 1:2,5 beträgt.

3. Verfahren nach Ansprüchen 1 und 2, dadurch gekennzeichnet, daß die Umsetzung bei Temperaturen im Bereich von 140 bis 180°C erfolgt.

4. Verfahren nach Ansprüchen 1 bis 3, dadurch gekennzeichnet, daß man das als Nebenprodukt gebildete Benzoylchlorid zusammen mit restlicher nichtumgesetzter Benzoesäure und restlichen nichtumgesetztem Benzotrichlorid vom Reaktionsgemisch abtrennt und dem Ausgangsreaktionsgemisch wieder zusetzt.

5. Verfahren nach Ansprüchen 1 bis 4, dadurch gekennzeichnet, daß ein Inertgas in das Reaktionsgemisch eingeleitet wird und/oder dem Reaktionsgemisch inerte organische Lösungsmittel zugegeben werden.

6. Verfahren nach Anspruch 5, dadurch gekennzeichnet, daß man als Inertgas Stickstoff verwendet.

7. Verfahren nach Anspruch 5, dadurch gekennzeichnet, daß man als inerte organische Lösungsmittel solche verwendet, die unter Reaktionsbedingungen verdampfbar sind.

8. Verfahren nach Anspruch 7, dadurch gekennzeichnet, daß man als inerte organische Lösungsmittel Chlorbenzol, Dichlorbenzol und/oder Dichlormethan verwendet.

### Revendications

1. Procédé de préparation de l'anhydride benzoïque, caractérisé en ce que l'on fait réagir le benzotrichlorure avec l'acide benzoïque dans un rapport molaire de 1:2 à 1:3 à des températures dans l'intervalle de 100 à 200°C.

2. Procédé selon la revendication 1, caractérisé en ce que le rapport molaire benzotrichlorure/acide benzoïque est de 1:2:5.

3. Procédé selon les revendications 1 et 2, caractérisé en ce que la réaction est effectuée à des températures dans l'intervalle de 140 à 180°C.

4. Procédé selon l'une quelconque des revendications 1 à 3, caractérisé en ce que le chlorure de benzoyle formé en produit secondaire est séparé avec l'acide benzoïque résiduel non converti et le benzotrichlorure résiduel non converti du mélange de réaction et recyclé dans le mélange de réaction initial.

5. Procédé selon l'une quelconque des revendications 1 à 4, caractérisé en ce que l'on injecte un gaz inerte dans le mélange de réaction et/ou on ajoute un solvant organique au mélange de réaction.

6. Procédé selon la revendication 5, caractérisé en ce que l'on utilise en tant que gaz inerte l'azote.

7. Procédé selon la revendication 5, caractérisé en ce que que l'on utilise des solvants organiques inertes vaporisables dans les conditions de la réaction.

8. Procédé selon la revendication 7, caractérisé en ce que l'on utilise en tant que solvant organique inerte le chlorobenzène, le dichlorobenzène et/ou le dichlorométhane.

### Claims

1. Process for the preparation of benzoic anhydride, characterised in that benzotrichloride is reacted with benzoic acid in a molar ratio of 1:2 to 1:3 at temperatures in the range from 100 to 200°C.

2. Process according to Claim 1, characterised in that the molar ratio of benzotrichloride to benzoic acid is 1:2.5.

3. Process according to Claims 1 and 2, characterised in that the reaction is carried out at temperatures in the range from 140 to 180°C.

4. Process according to Claims 1 to 3, characterised in that the benzoyl chloride formed as a by-product, together with residual unreacted benzoic acid and residual unreacted benzotrichloride, is separated off from the reaction mixture and added again to the starting reaction mixture.

5. Process according to Claims 1 to 4, characterised in that an inert gas is passed into the reaction mixture and/or inert organic solvents are added to the reaction mixture.

6. Process according to Claim 5, characterised in that nitrogen is used as the inert gas.

7. Process according to Claim 5, characterised in that inert organic solvents which are used are those which can be evaporated under the reaction conditions.

8. Process according to Claim 7, characterised in that chlorobenzene, dichlorobenzene and/or methylene chloride are used as the inert organic solvents.